# EUROPEAN PATENT APPLICATION

(11) **EP 3 257 514 A1**
(43) Date of publication of application: **20.12.2017**
(21) Application number: 16843285.4
(22) Date of filing: 04.05.2016
(51) Int. Cl.: A61K 31/704, A23K 20/163

(54) **ANTIDIABETIC EFFECT OF GYPENOSIDE 75**

(71) Applicant: Intelligent Synthetic Biology Center, Daejeon 34141 (KR)
(72) Inventor: KIM, Sun Chang, Daejeon 34141 (KR); PARK, Chan Bae, Gyeonggi-do 16975 (KR); LEE, Soon Jang, Gyeonggi-do16943 (KR)
(74) Representative: Krauss, Jan
(86) International application number: PCT/KR2016/004720
(87) International publication number: WO 2017/191856

(57) **Abstract**

The present invention relates to a composition for preventing, improving, or treating diabetes mellitus, and more specifically, relates to pharmaceutical, food, and feed compositions comprising gypenoside 75 or a pharmaceutically acceptable salt thereof Additionally, the present invention relates to a method for preventing or treating diabetes mellitus using the composition.

## Description

### [Technical Field]

The present invention relates to a pharmaceutical composition for preventing or treating diabetes mellitus, comprising gypenoside 75 or a pharmaceutically acceptable salt thereof; and more specifically relates to a method for preventing or treating diabetes mellitus, comprising administering the pharmaceutical composition for preventing or treating diabetes mellitus, comprising gypenoside 75 or a pharmaceutically acceptable salt thereof, to a non-human subject; a food composition for preventing or improving diabetes mellitus, comprising gypenoside 75 or a sitologically acceptable salt thereof; and a feed composition for preventing or improving diabetes mellitus, comprising gypenoside 75 or a sitologically acceptable salt thereof.

### [Background Art]

Diabetes mellitus, one of diseases that threaten human health, causes, as a chronic metabolic disease, vascular disorders and functional defects of nerve, kidney, retina, *etc.,* over a long period of time, thereby leading to death. Diabetes mellitus is broadly classified into insulin-dependent diabetes mellitus (type 1 diabetes) and non-insulin-dependent diabetes mellitus (type 2 diabetes) according to mechanisms by which they develop.

Insulin-dependent diabetes mellitus is known to be caused by the inability to consume blood glucose because pancreatic beta cells which are selectively damaged or destroyed cannot produce insulin in the body, and involves serious hyperglycemia, ketoacidosis, polydipsia, polyuria, weight loss, and fatigue. Insulin-dependent diabetes mellitus typically begins in children or teenagers, and its treatment method is administration of insulin.

Non-insulin-dependent diabetes mellitus accounts for more than 90% of diabetic patients and occurs mainly in adults. Causes of type 2 diabetes have not been clearly established. However, it is presumed that a decrease in insulin secretion by pancreatic beta cells and an increase in insulin resistance in tissues of the liver, muscle, fat, *etc.,* due to a combination of genetic factors and environmental factors such as abnormal diet, stress, exercise, obesity, aging, *etc.* are related to inducement of non-insulin-dependent diabetes mellitus. The non-insulin-dependent diabetes mellitus is generally resistant to insulin, and usually maintains a high blood glucose level due to impaired insulin action. As chronic hyperglycemia causes damage to the pancreatic beta cells, leading to cell death, it is necessary to regulate blood glucose levels effectively for treatment of type 2 diabetes, in which normal insulin action plays an important role.

Meanwhile, elderly people and young adults with similar physical conditions were compared in clinical trials to investigate causes of the insulin resistance displayed in the elderly. As a result, it was found that defects occurred in the glucose metabolism of the muscles of the elderly. Additionally, according to nuclear magnetic resonance analysis, the mitochondrial activities decreased in the muscles of the elderly. This result indicates that mitochondrial dysfunction in the muscle cells of the elderly may cause non-insulin-dependent diabetes mellitus. In fact, the expression of ATP synthase β, a mitochondrial protein, was found to decrease in the muscles of non-insulin-dependent diabetic patients (J Biol Chem, 278: 10436-10442 (2003)). That is, mitochondrial activation may also be an approach to the treatment for non-insulin-dependent diabetes mellitus.

Currently, methods for treating non-insulin-dependent diabetes mellitus include diet treatment, kinesiotherapy, pharmacotherapy, *etc.* As drugs used in the treatment of diabetes mellitus, various types of excellent hypoglycemic agents, such as sulfonylurea-, biguanide-, alpha-glucosidase inhibitor-, thiazolidinedione-, and meglitinide-types, are produced and commercially available. However, such drugs may have mild symptoms such as vomiting, abdominal pain, and abdominal distension (International Hepatology Communications, 5: 289-296 (1996)), and side effects such as hypoglycemia and decreased liver function (Diabetes Care 1995: 18(6): 817-824), and in a severe case, death (International Hepatology Communications, 5: 289-296(1996)). Further, those who are in a medical condition such as liver disease, gastrointestinal tract disorder, kidney disease, pregnancy, *etc.* are restricted in the use of the drugs.

Additionally, drugs based on metformin strongly activate AMP activated kinase (AMPK) which plays an important role in glucose metabolism during the gluconeogenesis pathway, thereby actively suppressing abnormal gluconeogenesis. Consequently, many pharmaceutical companies manufacture the drugs and make them available in the market. However, there are research reports that a large dose of metformin administration for a long period of time increases risk of vitamin B12 deficiency, and thus, it cannot be considered as a safe drug.

Accordingly, a large scale research has been conducted on natural products used as oriental medicine, folk remedies, *etc.* for development of a novel diabetes mellitus medicine and functional foods with outstanding pharmacological activity, little toxicity, and few side effects. Recently, researches have been conducted on prevention or treatment of diabetes mellitus, such as Korean Patent Application No. 2011-0020469 which discloses a composition for preventing or treating diabetes mellitus, which comprises ginsenoside Rg2 and Rg3.

Meanwhile, ginseng contains various types of ginsenoside and gypenoside, such as panaxadiol (PD), panaxatriol (PT), and oleanane types. As the different types have different functions such as enhancement of immune function, excitement of the central nervous system, stimulation of adrenocorticotropic hormone secretion, inhibition of platelet aggregation, *etc.,* it is difficult to predict the same effect even among ginsenosides of the same types.

### [Disclosure]

### [Technical Problem]

An object of the present invention is to provide a pharmaceutical composition for preventing or treating diabetes mellitus, comprising gypenoside 75 or a pharmaceutically acceptable salt thereof.

Another object of the present invention is to provide a method for preventing or treating diabetes mellitus, comprising administering the pharmaceutical composition to a non-human subject.

Still another object of the present invention is to provide a food composition for preventing or improving diabetes mellitus, comprising gypenoside 75 or a sitologically acceptable salt thereof.

Still another object of the present invention is to provide a feed composition for preventing or improving diabetes mellitus, comprising gypenoside 75 or a sitologically acceptable salt thereof.

### [Technical Solution]

The present inventors have made diligent research efforts in order to develop a method for preventing or treating diabetes mellitus. As a result, the present inventors have completed the present invention by confirming that gypenoside 75 stimulates insulin secretion of insulin-secreting cells as well as activating mitochondrial functions, thereby showing a prophylactic or therapeutic effect on diabetes mellitus.

### [Advantageous Effects]

The composition according to the present invention, which comprises gypenoside 75, stimulates insulin secretion and increases mitochondrial activity, thereby showing the preventing and treating effects on diabetes mellitus.

### [Description of the Drawings]

Fig. 1 shows the results of ELISA quantitative analysis of the amount of insulin secreted by administration of gypenoside 75.
Fig. 2 shows the analysis results of luciferase activity analysis for the amount of intracellular ATP increased by administration of gypenoside 75.
Fig. 3 shows the results of tetramethylrhodamine methyl ester (TMRM) staining and fluorescence activated cell sorting (FACS) analysis for mitochondrial membrane potentials increased by administration of gypenoside 75.

### [Best Mode]

As an aspect to achieve the above objects, the present invention provides a pharmaceutical composition for preventing or treating diabetes mellitus, comprising gypenoside 75 or a pharmaceutically acceptable salt thereof.

Gypenoside 75 of the present invention may preferably be gypenoside 75 having the structure of Formula 1 below:

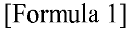

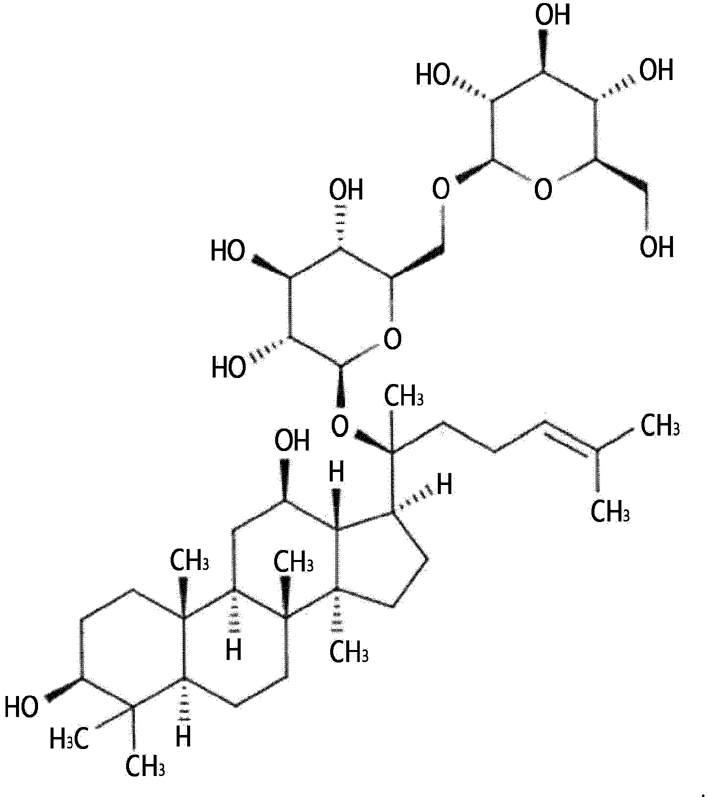

The gypenoside 75 can be chemically synthesized or isolated from natural substances. In a case where gypenoside 75 is isolated from a natural substance, as long as gypenoside 75 is contained, the isolated substance may include all of the extracts of the natural substance or fractions thereof

As used herein, the term "pharmaceutically acceptable salt" means a formulation that does not damage biological activities and properties of administered gypenoside 75. The pharmaceutically acceptable salts, unless otherwise indicated, may include all of acidic or basic salts that may exist in compounds of the gypenoside 75. For example, the pharmaceutically acceptable salts may include sodium, calcium, and potassium salts of a hydroxy group, and other pharmaceutically acceptable salts may include hydrobromide, sulfate salt, hydrogen sulfate salt, phosphate salt, hydrogen phosphate salt, dihydrogen phosphate salt, acetate, succinate, citrate, tartrate, lactate, mandelate, methanesulfonate (methylate), and p-toluenesulfonate (tosylate) salts of an amino group. The pharmaceutically acceptable salt may be prepared by salt preparation methods known in the art.

As used herein, the term "prevent" means all behaviors that inhibit or delay diabetes mellitus by administering the composition to a subject.

As used herein, the term "treat" means all behaviors that advantageously change or improve symptoms of diabetes mellitus by administering the composition to a subject.

The pharmaceutical composition of the present invention has an excellent prophylactic or therapeutic effects on diabetes mellitus due to gypenoside 75 or a pharmaceutically acceptable salt thereof. Additionally, as it is non-toxic, the pharmaceutical composition is safe from side effects when administered to humans.

The pharmaceutical composition of the present invention may be used as a single formulation, or may be prepared and used as a complex formulation, which further comprises a drug known to have an authorized therapeutic effect on diabetes mellitus. Additionally, by formulating using a pharmaceutically acceptable carrier or excipient, it can be prepared in a unit dose form or encapsulated into a multi-dose container.

The pharmaceutical composition of the present invention may be formulated and used in the form of oral formulation such as powder, granule, pill, capsule, suspension, emulsion, syrup, aerosol, *etc.,* topical formulation, or sterile injection solution according to each preparation method. Carriers, excipients, and diluents that can be included in the composition include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybeonzoate, talc, magnesium stearate, mineral oil, *etc.,* but are not limited thereto. They can be used independently or in combination of two or more.

When the pharmaceutical composition of the present invention is formulated, conventionally used diluents or excipients such as filling agents, extending agents, binding agents, wetting agents, disintegrants, surfactants, *etc.* can be used to prepare the composition.

Examples of solid preparations for oral administration may include tablets, pills, powder, granules, capsules, *etc.,* and can be prepared by mixing one or more excipients such as starch, calcium carbonate, sucrose, lactose, and gelatin. In addition to simple excipients, a lubricant such as magnesium stearate and talc may also be used. Examples of liquid preparations for oral administration may include suspensions, liquid medicine for internal use, emulsions, syrups, *etc.,* in which various excipients such as humectants, sweeteners, flavoring agents, and preservatives. are included in addition to water and liquid paraffin which are frequently-used simple diluents.

Parenteral preparations may include sterilized aqueous solutions, non-aqueous solvents, suspensions, emulsions, lyophilized preparations, suppositories, *etc.* The non-aqueous solvents and suspensions may include propylene glycol, polyethylene glycol, vegetable oils such as olive oil, injectable esters such as ethyl oleate, *etc.*

A pharmaceutically effective amount for administration of the pharmaceutical composition of the present invention may vary depending on a formulation method, administration method, administration time and/or administration route, *etc.,* as well as various factors such as a type and degree of response to be achieved by administration, types of subjects to be administered, age, body weight, general health condition, symptoms or severity of a disease, gender, diet, excretion, and ingredients of other drugs administered simultaneously or at a different time, *etc*.; and similar factors well-known in the art. One of ordinary skill in the art can easily determine and prescribe an effective dose for the intended treatment.

The pharmaceutical composition of the present invention may comprise gypenoside 75 in a pharmaceutically effective amount.

As used herein, the term "pharmaceutically effective amount" refers to an amount sufficient to treat diseases, at a reasonable benefit/risk ratio applicable to any medical treatment. Generally, an amount of 0.001 mg/kg to 1,000 mg/kg, preferably 0.05 mg/kg to 200 mg/kg, more preferably 0.1 mg/kg to 100 mg/kg may be administered once a day or in divided doses multiple times a day. In view of the purpose of the present invention, however, it is preferable that a specific therapeutically effective amount is administered to a particular patient depending on various factors such as type and degree of response to be achieved, a specific composition including other drugs if there are any, patient's age, body weight, general health condition, gender, diet, administration time, administration route, secretion rate of composition, duration of treatment, and drugs administered in combination or simultaneously with the specific composition; and other similar factors well-known in the art.

The content of the extract included in the pharmaceutical composition may be in a range of 0.01 wt% to 100 wt%, more preferably 1 wt% to 80 wt%, relative to the total weight, but is not particularly limited thereto.

The pharmaceutical composition of the present invention may be administered as an independent drug or in combination with other drugs, and may be administered sequentially or simultaneously with conventional drugs in a single or multiple doses. Considering all of the factors described above, it is important to administer a minimum amount that can have a maximum therapeutic effect without causing any side effect. This can be easily determined by one of ordinary skill in the art.

An administration route and administration method of the pharmaceutical composition of the present invention may each be independent, and are not particularly limited. Also, as long as the pharmaceutical composition can be delivered to a target tissue, it can be administered by any administration route and administration method. The pharmaceutical composition can be administered orally or parenterally, preferably parenterally.

As the parenteral administration method, for example, intravenous administration, intraperitoneal administration, intramuscular administration, transdermal administration, subcutaneous administration, *etc.* can be used.

According to another aspect, the present invention provides a pharmaceutical composition further comprising a pharmaceutically acceptable carrier.

As used herein, "pharmaceutically acceptable carrier" may refer to a carrier or diluent that neither causes stimulation to an organism nor abolishes biological activities or properties of a compound to be administered thereto. Types of carriers that can be used in the present invention are not particularly limited, and any carrier may be used as long as it is conventionally used in the art and is pharmaceutically acceptable. As non-limiting examples of the carrier, a saline solution, sterile water, Ringer's solution, buffered saline, an albumin injection solution, a dextrose solution, a maltodextrin solution, glycerol, ethanol, *etc.* may be used. The carrier can be used independently or in combination of two or more. The carrier may comprise a non-naturally occurring carrier.

Additionally, if necessary, other conventional additives such as an antioxidant agent, a buffer solution, and/or a bacteriostatic agent, may be further added and used, and the composition may be formulated into a injection formulation such as an aqueous solution, suspension, emulsion; pills; capsules; granules; or tablets, by additionally adding a diluent, dispersant, surfactant, binding agent, lubricant, *etc.*

According to another aspect, the present invention provides a pharmaceutical composition for preventing or treating diabetes mellitus, wherein the diabetes mellitus, a subject disease to be prevented or treated by the pharmaceutical composition, is non-insulin-dependent diabetes mellitus.

As used herein, "non-insulin-dependent diabetes mellitus", also called type 2 diabetes mellitus, occurs when insulin is secreted, but either the secreted insulin is not enough or the body cannot use it effectively. Non-insulin-dependent diabetes mellitus accounts for 90 to 95 percent of all cases of diabetes mellitus, and it usually develops after the age of 40. However, the age of onset is recently becoming lower, and it is now being seen in children as well. Symptoms of the non-insulin-dependent diabetes mellitus may include extreme thirst, frequent need to urinate, fatigue, and increased feeling of hunger.

According to another aspect, the present invention provides a method for preventing or treating diabetes mellitus, comprising administering a pharmaceutical composition for preventing or treating diabetes mellitus, comprising the gypenoside 75 or a pharmaceutically acceptable salt thereof, to a non-human subject.

In view of the method for preventing or treating the diabetes mellitus of the present invention, the pharmaceutical composition is the same as the pharmaceutical composition of the present invention described above.

As used herein, the term "subject" refers to all mammalian animals including humans, mice, livestock, *etc.*

In view of the prevention and treatment methods of the present invention, an amount, route, and method of administration are the same as those of the pharmaceutical composition of the present invention described above.

In view of the prevention and treatment methods of the present invention, the pharmaceutical composition can be administered orally or parenterally without any particular limitation.

According to another aspect, the present invention provides a food composition for preventing or improving diabetes mellitus, comprising gypenoside 75 or a sitologically acceptable salt thereof.

Gypenoside 75 of the present invention may preferably be gypenoside 75 having the structure of Formula 1 below:

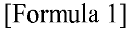

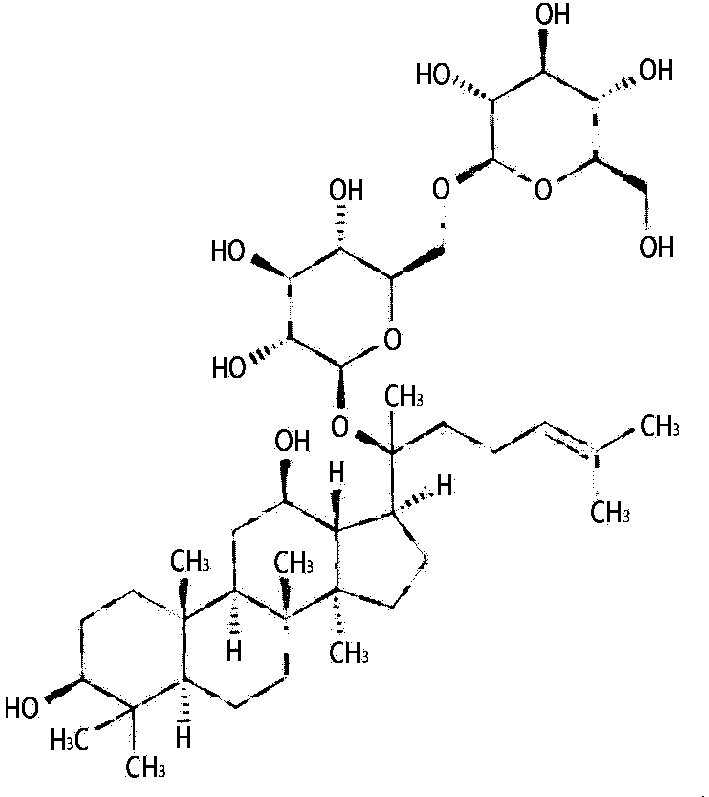

Gypenoside 75 or a sitologically acceptable salt thereof included in the food composition of the present invention may be included in the form of an animal or plant, an extract thereof, a fraction thereof, or a processed product thereof, comprising gypenoside 75. Additionally, the composition may comprise a sitologically acceptable food additive.

As used herein, the term "food additive" refers to an ingredient that can be added subsidiarily to a food. As an additive added in preparation of each formulation of health functional foods, one of ordinary skill in the art can appropriately select and use it. Examples of the food additives include various nutritional supplements, vitamins, minerals (electrolytes), flavors such as synthetic and natural flavors, coloring agents, filling agents, pectic acid and a salt thereof, alginic acid and a salt thereof, organic acid, protective colloid thickening agents, pH adjusters, stabilizers, preservatives, glycerin, alcohol, and a carbonating agent used in carbonated beverages, *etc.,* but types of the food additives are not limited by the examples.

The food composition of the present invention may include a health functional food. As used herein, "health functional food" refers to a food prepared or processed in the form of a tablet, a capsule, powder, a granule, liquid, and a pill using raw materials or ingredients that have useful functional properties for the human body. As used herein, the term "functional" refers to adjusting nutrients for structures and functions of the human body and obtaining useful effects for health use such as physiological action. The health functional foods of the present invention may be manufactured by methods conventionally used in the art, and when they are prepared, raw materials and ingredients which are conventionally added in the art can be added for preparations. Additionally, types of formulations of the health functional foods may be manufactured without limitation as long as they are acknowledged as health functional foods. The food composition of the present invention can be manufactured in various types of formulations. Additionally, in contrast to conventional drugs, it is advantageous that there is no side effect caused by a long-term administration as the food composition uses foods as raw materials. Additionally, the health functional food of the present invention is portable, and thus can be consumed as a supplement for promoting the prophylactic and improvement effect on diabetes mellitus.

There are no restrictions on the form that the health functional food of the present invention can take, and it can contain all conventional foods and can be used interchangeably with terms known in the art such as functional foods. In addition, the health functional food comprising gypenoside 75 of the present invention can be prepared by mixing with other suitable supplement ingredients and additives known in the art that can be contained in the food, which are selected by one of ordinary skill in the art. Examples of the foods that can be added include meat, sausages, bread, chocolate, candies, snacks, pizza, ramen noodles, other noodles, gums, dairy products such as ice cream, various soups, beverages, tea, drinks, alcohols, and vitamin complex, *etc.* Concentrates, tea, jellies, and juice, which contain gypenoside 75 of the present invention as the main ingredient can also be added in preparation of the health functional foods.

In particular, the content of the extract included in the food composition may be in a range of 0.01 wt% to 100 wt%, more preferably 1 wt% to 80 wt%, relative to the total weight of the food composition, but is not limited thereto.

According to another aspect, the present invention provides a feed composition for preventing or improving diabetes mellitus, comprising gypenoside 75 or a pharmaceutically acceptable salt thereof.

Gypenoside 75 of the present invention may preferably be gypenoside 75 having the structure of Formula 1 below:

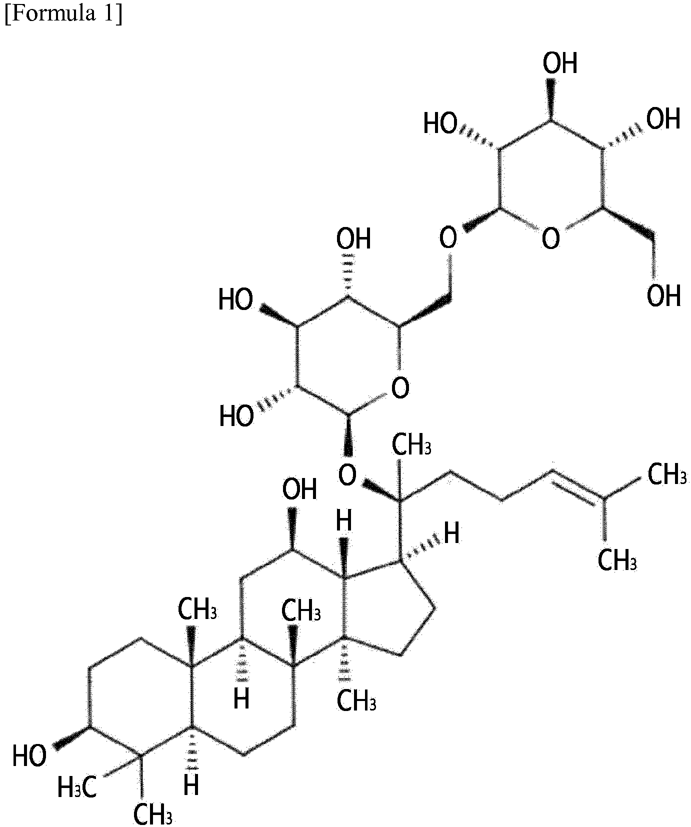

The feed composition of the present invention may be a feed composition for vertebrates such as cattle and pigs, poultry such as chickens and ducks, fish, crustaceans, *etc.,* but is not limited thereto. It is obvious that as long as animals can intake gypenoside 75, the present invention can be practiced with those animals.

The feed composition of the present invention can be used regardless of the mixing ratio within the feed composition. For example, even a feed which contains a large amount of protein can be used in the present invention without difficulty.

In particular, the content of the extract included in the feed composition may be in a range of 0.01 wt% to 100 wt%, more preferably 1 wt% to 80 wt%, relative to the total weight of the food composition, but is not limited thereto.

The feed composition of the present invention may include grains such as corns, husks such as wheat brans, broken rice grains and cornhusk, meals such as soybean meal and perilla meal, , root plants such as sweet potatoes and potatoes, food process byproducts such as bread byproducts and amino acid fermentation byproducts, algae such as chlorella and sea mustard, fibers such as grasses and mountain grasses and fermented products thereof, medicine byproducts such as ginseng meal and inositol meal, oils such as corn oil, soybean oil, beef tallow, lard, and fish oil, starches such as grain starch, proteins such as fish flour and crab flour, inorganic substances such as bone meal, fishbone meal, and shellfish fragment, table salts such as mineral salts and bay salts, phosphate salts, calcium salts, *etc.*

In order to prevent deterioration of the quality of the feed, the feed composition of the present invention may comprise binding agents such as guar gum, resin, gelatin, casein, sodium alginate, and sodium casein, lubricants such as sucrose fatty acid ester, and sorbitan fatty acid ester, acidifiers such as citric acid lactic acid, anticoagulants such as white carbon, and antioxidants such as ethoxyquin and butylhydroxytoluene.

The feed composition of the present invention may further comprise enzymes such as amino acids, vitamins, and glycolytic enzymes, probiotics such as *Lactobacillus reuteri*, and yeasts such as beer yeast.

The feed composition of the present invention is not limited in terms of its formulation, and any commonly used feed composition can be used in the present invention. For example, the feed composition may be liquid, powder, a granule, or a pellet.

### [Mode for Invention]

Hereinbelow, the constitution and effects of the present invention will be described in detail with accompanying exemplary embodiments. However, the exemplary embodiments disclosed herein are only for illustrative purposes and should not be construed as limiting the scope of the present invention.

### Example 1: Effect of gypenoside 75 on increase in insulin secretion

In order to confirm the effect of gypenoside 75 on an increase in insulin secretion, the amount of insulin secreted after treatment with gypenoside 75 was measured.

Specifically, in order to confirm the effect of gypenoside 75 on an increase in insulin secretion, INS-1 cells, which are insulin-secreting cells, were cultured in a cell incubator (37°C, 5% CO₂, RPMI culture medium, 10% FBS). The cells were treated with each of 10 µM of gypenoside 75, ginsenoside F1, ginsenoside Rg3, ginsenoside Rb2, and ginsenoside Rb3 for 2 hours, and the amounts of insulin secreted after the treatments were then measured via the ELISA method.

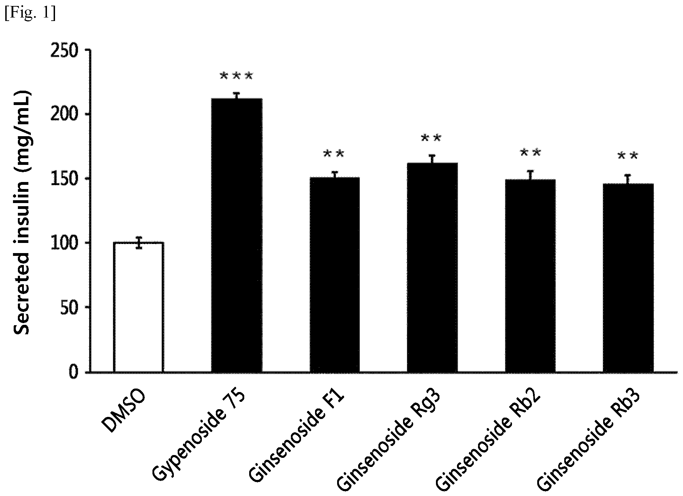

As a result, as shown in Fig. 1, when gypenoside 75 was treated, the amount of insulin secreted increased by about 110% compared to that of the control group (DMSO). Accordingly, it was known that an increase of insulin secretion due to treatment with gypenoside 75 can lead to prevention or treatment of diabetes mellitus. It was also confirmed that ginsenoside F1, ginsenoside Rg3, ginsenoside Rb2, and ginsenoside Rb3 had effects on an increase of insulin secretion by 50%.

### Example 2: Activation of mitochondrial function of gypenoside 75

If mitochondrial function is impaired, it may cause diabetes mellitus by interfering with insulin signaling. Accordingly, in order to confirm the effect of gypenoside 75 on the activation of the mitochondrial function, after gypenoside 75 was treated, an increased amount of ATP and mitochondrial membrane potentials were measured.

Specifically, in order to confirm the effect of gypenoside 75 on the activation of the mitochondrial function, INS-1 cells, which are insulin-secreting cells, were cultured in a cell incubator (37°C, 5% CO₂, RPMI culture medium, 10% FBS). The cells were treated with 10 µM of gypenoside 75 for 2 hours, and the amount of ATP and mitochondrial membrane potentials inside the cells were then measured. To measure the amount of intracellular ATP, the INS-1 cells treated with gypenoside 75 were lysed through freezing-thawing and ultrasonication methods. The cell lysates were mixed with luciferase and luciferin to induce fluorescence. As the activation of the luciferase and fluorescence induced thereby depend on the amount of ATP, the degree of fluorescence was measured with a fluorometer to determine the amount of ATP. To measure the mitochondrial membrane potential, the INS-1 cells treated with gypenoside 75 were then stained with TMRM, and the degree of the staining of each cell was measured using a FACS instrument. As the mitochondrial membrane potential increases, the degree of staining by TMRM increases.

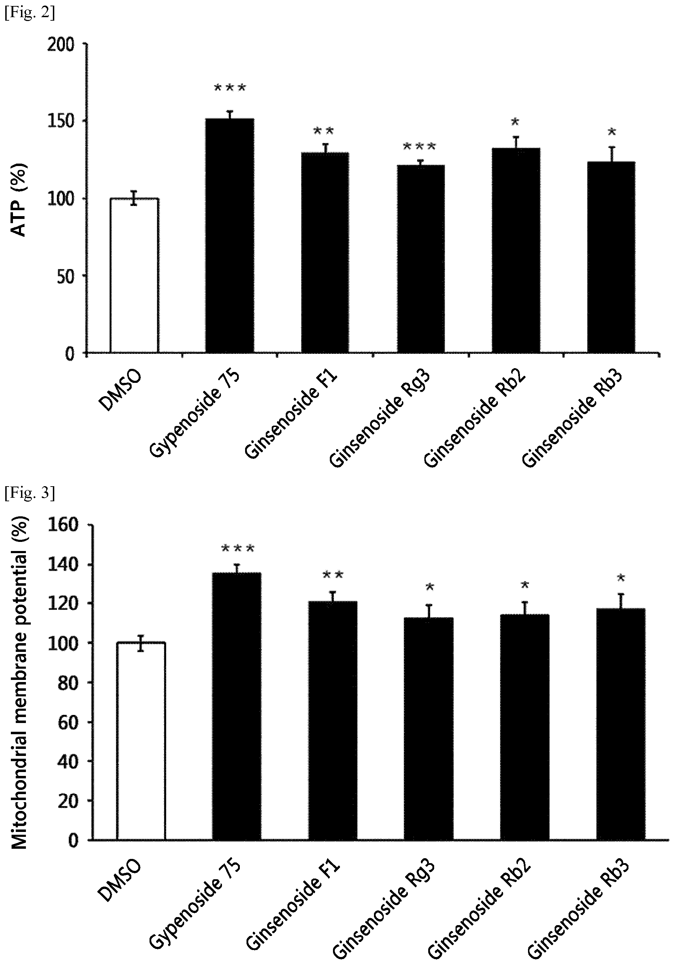

As a result, as shown in Fig. 2, when gypenoside 75 was treated, the amount of intracellular ATP was increased by 50% compared to that of the control group (DMSO). Additionally, it was confirmed that ginsenoside F1, ginsenoside Rg3, ginsenoside Rb2, and ginsenoside Rb3 have an effect of increasing the amount of ATP by about 40%. As shown in Fig. 3, when gypenoside 75 was treated, the mitochondrial membrane potential was increased by about 40% compared to that of the control group (DMSO). Additionally, it was also confirmed that ginsenoside F1, ginsenoside Rg3, ginsenoside Rb2, and ginsenoside Rb3 had an effect of increasing the mitochondrial membrane potential by about 20%.

Such results show that when cells are treated with gypenoside 75, the mitochondrial function is activated, which increases ATP synthesis and mitochondrial membrane potentials. Consequently, it was confirmed that diabetes mellitus can be prevented or treated by the effect of activating mitochondrial functions upon the treatment of gypenoside 75.

## Claims

1. A pharmaceutical composition for preventing or treating diabetes mellitus, comprising gypenoside 75 or a pharmaceutically acceptable salt thereof.

2. The pharmaceutical composition of claim 1, wherein gypenoside 75 is represented by Formula 1 below:

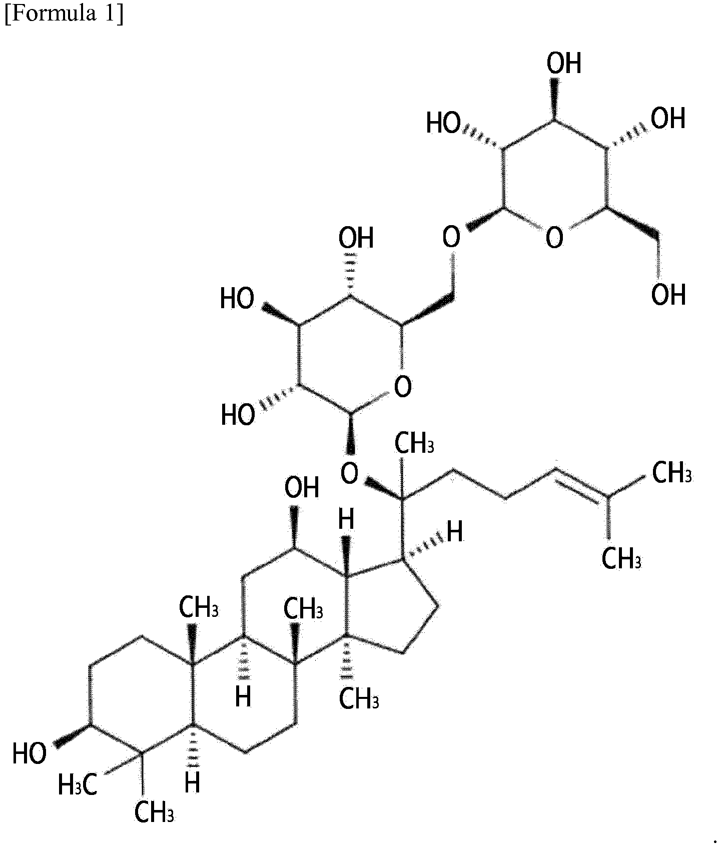

3. The pharmaceutical composition of claim 1, wherein the composition further comprises a pharmaceutically acceptable carrier.

4. The pharmaceutical composition of claim 1, wherein the diabetes mellitus is non-insulin-dependent diabetes mellitus.

5. A method for preventing or treating diabetes mellitus comprising administering any pharmaceutical composition of claims 1 to 4 to a non-human subject.

6. A food composition for preventing or improving diabetes mellitus, comprising gypenoside 75 or a sitologically acceptable salt thereof.

7. The food composition of claim 6, wherein gypenoside 75 is represented by Formula 1:

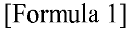

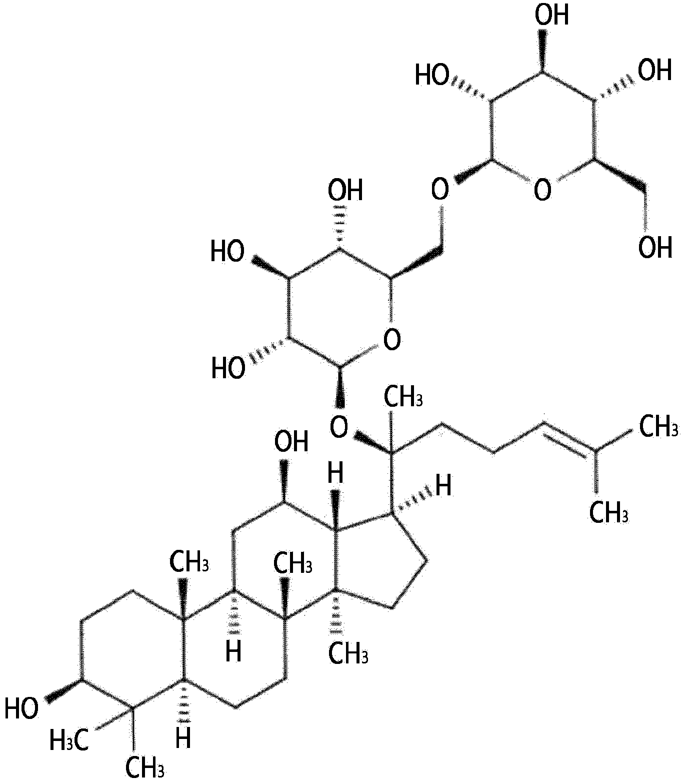

8. A feed composition for preventing or improving diabetes mellitus, comprising gypenoside 75 or a pharmaceutically acceptable salt thereof

9. The feed composition of claim 8, wherein gypenoside 75 is represented by Formula 1
